# EUROPEAN PATENT APPLICATION

(11) **EP 2 087 923 A1**
(43) Date of publication of application: **12.08.2009**
(21) Application number: 08381005.1
(22) Date of filing: 11.02.2008
(51) Int. Cl.: A61N 1/32, A61N 1/30

(54) **Electrotherapy machine**

(71) Applicant: Font I Mas, Juan Carlos, 08850 Gava (ES)
(72) Inventor: Font I Mas, Juan Carlos, 08850 Gava (ES)
(74) Representative: Esteban Perez-Serrano, Maria Isabel

(57) **Abstract**

This invention relates to an electrotherapy machine, specifically used in order to increase the absorption of cosmetic products through the skin, so that the cosmetic products applied reach deep layers, increasing the effect thereof. The machine which is the object of the invention comprises a first pole of a pair of electrodes located on a head, it being possible to move this over the patient's skin, and a second pole of the pair of electrodes which closes the electrical circuit on the patient's body. The machine which is the object of the invention is **characterised in that** the electrical signal emitted by one of the poles comprises a wave with voltage pulses featuring a DC component so that the pulsing wave carries out an electroporation effect and the DC component an electrophoretic effect.

## Description

### OBJECT OF THE INVENTION

This invention relates to an electrotherapy machine, specifically used in order to increase the absorption of cosmetic products through the skin, so that the cosmetic products applied reach deep layers, thus increasing the effect thereof.

The machine which is the object of the invention comprises a first pole of a pair of electrodes, located on a head which may be moved over the patient's skin, and the second pole of the pair of electrodes which closes the electrical circuit on the patient's body.

The machine which is the object of the invention is characterised in that the electrical signal emitted by one of the poles comprises a wave with voltage pulses which feature a DC component, so that the pulsing wave executes an electroporation effect and the DC component an electrophoretic effect.

With this type of signal emitted, it is achieved that on the one hand the voltage pulses bring about an electroporation effect, causing the opening of the pores of the skin, increasing the absorption of the cosmetic product applied, and generating between the two poles, by means of the DC component, a current which ionises the cosmetic, on the condition that this is ionisable, favouring the transport thereof through the skin. Both physical phenomena are carried out simultaneously thanks to the type of wave generated by the electrotherapy machine which is the object of this invention, minimising the risk of damage to the patient's skin.

### BACKGROUND OF THE INVENTION

Electrotherapy machines which produce an effect of electrophoresis or iontophoresis are well known. They normally feature at least one pair of electrodes, applying one pole to the area of the body to be treated and the other pole closing the direct current electrical circuit through the patient's body. Although an increase in the penetration of the cosmetic into the patient's skin, in the guise of ionic molecules, is achieved by means of these machines, it is necessary to increase the intensity in order to increase the degree of penetration, which may bring about adverse effects on the skin treated, such as burns, for example, due to the fact that electrophoresis can cause cutaneous lesions to the skin due to the electrolysis of the sodium chloride caused by the electric current, this undesirable effect increasing with the intensity and time of exposure.

Electrotherapy machines which bring about an electroporation effect consisting of the opening of small pores in the skin by means of the application of pairs of electrodes located close together and to which voltage surges are applied, which achieve the opening of the aforementioned pores, are also well known. By means of controlling the voltage applied and the time of exposure, the formation of these pores is reversible.

Machines which carry out both functions alternately, i.e., first a current capable of bringing about electroporation, and subsequently a current capable of bringing about electrophoresis, are also well-known.

Electrotherapy machines which can carry out both operations simultaneously, thanks to the form of the outgoing wave used, are unknown. Therefore, the electrotherapy machine carries out a function similar to traditional iontophoresis, but in a more efficient, safer way, minimising the risk of burns and using a single pair of electrodes. The electrotherapy machine which is the object of this invention uses a current of a very low intensity in order to avoid undesired effects; this, combined with the sinusoid electroporation wave, does not entail any risk and achieves a deep penetration of the cosmetics applied to the cutaneous tissue.

### DESCRIPTION OF THE INVENTION

The electrotherapy machine which is the object of this invention is used in order to increase the absorption of cosmetic products through the skin, so that the cosmetic products applied reach deep layers, increasing the efficiency thereof.

The equipment which is the object of this invention is of the electrophoroporator type, due to the fact that it simultaneously carries out the functions which include the principles of both electrophoresis and electroporation.

Electroporation consists of the application of brief voltage impulses on the skin, which bring about changes in the molecules, which act as small pores. Consequently, pores or channels appear in the molecular order. This effect produces an increase in permeability, improving the diffusion of the substances into the skin.

Electrophoresis or iontophoresis consists of the transportation of substances through the skin in the form of ions, using electric current at the application area. The equipment which is the object of this invention comprises a pair of poles, and the voltage generated at the positive pole repels the positive ions towards the negative pole, thrusting them through the cutaneous barrier.

The electrotherapy equipment which is the object of this invention introduces the electric current into the skin by means of one of the poles of the pair of electrodes, while a second pole closes the electrical circuit through the user. For this reason, it features only one pair of poles which carry out both the iontophorosis and the electroporation effects in accordance with the type of outgoing electrical signal.

One of the poles of the equipment would be located on a head which may be moved over the skin to be treated.

The electrotherapy machine which is the object of this invention is characterised in that the electrical signal emitted by one of the poles comprises a wave with voltage pulses, which features a DC component so that the pulsing wave carries out an electroporation effect and the DC component an electrophoretic effect.

### DESCRIPTION OF THE DRAWINGS

This specification is complemented by a plan which illustrates the preferred embodiment of the invention in a non-limitative way.

Figure 1 is an embodiment of the outgoing wave at the electric current emitting pole.

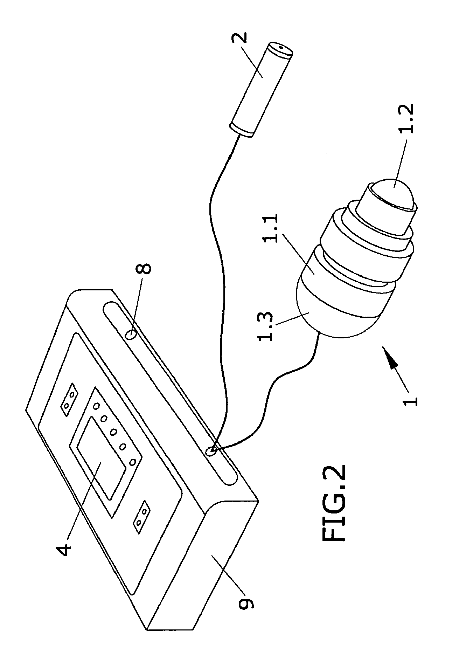

Figure 2 is an embodiment of the components of the electrotherapy machine which is the object of the invention.

Figure 3 is another embodiment of the components of the electrotherapy machine which is the object of the invention.

Figure 4 is an example of a block diagram of the general description of the equipment.

Figure 5 is a block diagram of the output stage.

### PREFERRED EMBODIMENT OF THE INVENTION

In the example of the embodiment, the first pole (1) of the pair of electrodes is embodied on a head (1.1) which features a "roll-on" type device (1.2) which slides over the patient's body and which corresponds to the output of the signal emitted.

The emitting pole (1) must have the same charge as the cosmetic substance to be applied. The cosmetic itself may include the conductive electrolyte.

The second pole (2) of the pair of electrodes, which closes the electrical circuit on the patient's body, corresponds to a rod which may be held in one of the patient's hand.

The signal emitted is of a low frequency.

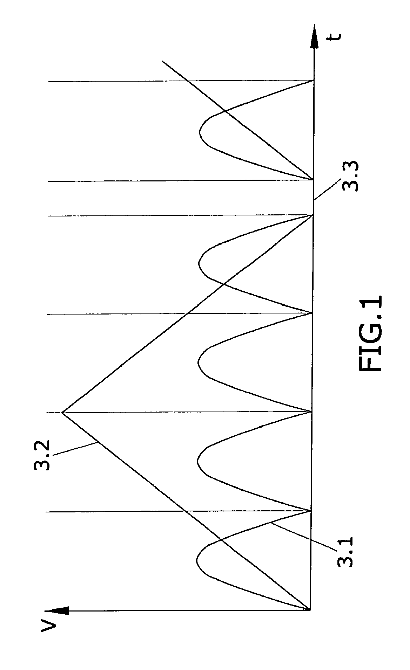

Figure 1 portrays an example of an outgoing wave from the equipment which is the object of this invention. A voltage-against-time diagram has been depicted in which the pulsing current generated (3.1) may be observed; this is surrounded by a DC component (3.2) in the form of a modulated wave which surrounds the voltage pulses (3.1). The fact that the wave is modulated prevents alterations to the pH of the skin.

The voltage surges applied are such that the phenomenon of pore formation is reversible.

The electrotherapy machine which is the object of the invention may feature different application programs in accordance with the different treatments to be applied, for example, firming, anti-cellulitic, etc. In order to do this, the user may change the frequencies, magnitudes and voltages for different types of treatment. The direct current will be constant for each program, with the pulse trains being variable.

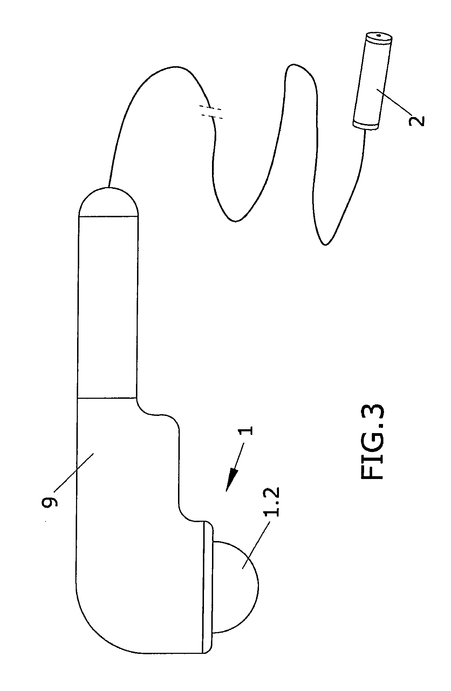

Figure 3 portrays an embodiment of the block diagram of the electrotherapy machine, which comprises a display or screen (4) which displays the different options of the programs performed thereby, a processing unit (5), a keyboard (6) for the inputting of data and for menu selection, and a power regulator (7) which controls the output levels, generating the modulation of the wave, and an output (8) which amplifies and shapes the signal starting from square wave impulses from the processing unit (5).

A power source transforms the alternating current from the conventional electrical network into the direct current used by the machine which is the object of the invention.

Part of the aforementioned elements may be integrated into a casing (9) of the type portrayed in the figures.

In order to emit the electrical signal, the processing unit (5) generates square pulses which supply the output stage (8).

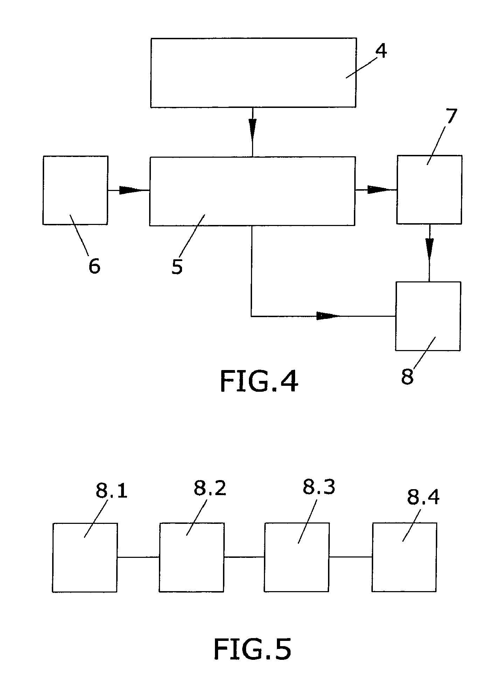

Figure 5 portrays a preferred embodiment of the output stage (8) which comprises a power amplifier (8.1) which amplifies the voltage and intensity of the signal received.

The amplifier (8.1) supplies a transformer (8.2) which raises the voltage and converts said voltage into alternating current. Subsequently, a rectifier (8.3) and a filter (8.4) transform the wave into a pulsing wave (3.1) with a modulated DC component (3.2), the modulation being depicted in the embodiment by means of a triangular wave.

With the purpose of allowing the skin to rest between consecutive pulse trains, there exists a period of inactivity (3.3).

In the embodiments portrayed, the head which carries the emitting pole
(1) comprises a means for the supply of the cosmetic element. It may, for example, feature a means for the insertion of a bottle (1.3) containing the product to be applied.

The machine may comprise two pairs of poles identical to those described, so that two treatments may be applied simultaneously on the patient's body.

## Claims

1. An electrotherapy machine, which comprises a first pole (1) of a pair of electrodes located on a head (1.1), it being possible to move this over the patient's skin, and a second pole (2) of the pair of electrodes which closes the electrical circuit on the patient's body, **characterised in that** the electrical signal emitted by one of the poles (1, 2) comprises a wave with a pulsing current (3.1) featuring a DC component (3.2) so that the pulsing wave (3.1) carries out an electroporation effect and the DC component (3.2) an electrophoretic effect.

2. An electrotherapy machine, according to claim 1, **characterised in that** the DC component (3.2) is modulated.

3. An electrotherapy machine, according to claim 1, **characterised in that** the means for the emission of the electrical signal generated comprise a processing unit (5) which generates square pulses which supply an output stage (8).

4. An electrotherapy machine, according to claim 3, **characterised in that** the output stage (8) comprises a power amplifier (8.1) which amplifies the voltage and intensity of the signal received, and which supplies a transformer (8.2) which raises the voltage and converts said voltage into alternating current, and subsequently, a rectifier (8.3) and a filter (8.4) transform the wave into a pulsing wave (3.1) with a DC component (3.2).

5. An electrotherapy machine, according to claim 1, **characterised in that** there is a period of inactivity (3.3) between consecutive pulse trains.

6. An electrotherapy machine, according to claim 1, **characterised in that** the pole (1) of the pair of electrodes located on the head (1.1) comprises a "roll-on" type element (1.2).

7. An electrotherapy machine, according to claim 1, **characterised in that** the head (1.1) comprises a means for the supply of a cosmetic element.

8. An electrotherapy machine, according to claim 1, **characterised in that** the means for the supply of a cosmetic element consists of a compartment for a bottle (1.3) containing said cosmetic element.

9. An electrotherapy machine, according to claim 1, **characterised in that** it comprises two pairs of poles (1, 2), so that two treatments may be applied simultaneously on the patient's body.
